# EUROPÄISCHE PATENTANMELDUNG

(11) **EP 1 095 986 A1**
(43) Veröffentlichungstag der Anmeldung: **02.05.2001**
(21) Anmeldenummer: 00120602.8
(22) Anmeldetag: 21.09.2000
(51) Int. Cl.: C09B 61/00, A23L 1/275, B01F 3/08, B01F 17/00

(54) **Verfahren zur Stabilisierung von flüssigen, wässrigen Präparaten fettlöslicher Substanzen**

(30) Priorität: 26.10.1999 DE 19951615
(71) Anmelder: BASF AKTIENGESELLSCHAFT, 67056 Ludwigshafen (DE)
(72) Erfinder: Runge, Frank, Dr., 67159 Friedelsheim (DE); Schweikert, Loni, Dr., 67122 Altrip (DE)

(57) **Zusammenfassung**

Verfahren zur Stabilisierung von flüssigen, wäßrigen Präparaten fettlöslicher Substanzen, dadurch gekennzeichnet, daß man
a) eine schutzkolloidfreie o/w-Emulsion, enthaltend 1 bis 50 Gew.-%, bezogen auf die Gesamtmenge der o/w-Emulsion mindestens einer fettlöslichen Substanz mit mindestens einem Schutzkolloid versetzt und
b) diese Zubereitung mit einer weiteren wäßrigen Phase vermischt.

## Beschreibung

Die vorliegende Erfindung betrifft ein Verfahren zur Stabilisierung von flüssigen, wäßrigen Präparaten fettlöslicher Substanzen.

Bei der Herstellung und Lagerung komplexer disperser Systeme wie Lebensmittelzubereitungen, beispielsweise Getränke oder Getränkekonzentrate, aber auch bei kosmetischen Emulsionen wie Cremes kann es zu kolloidalen Instabilitäten kommen.

Die Ursache dafür liegt in Wechselwirkungen zwischen Inhaltsstoffen und dispersen Phasen wie Emulsionen und Suspensionen, die destabilisierend wirken. Typische Inkompatibilitäten entstehen durch pH-Effekte und zu hohe Konzentrationen an neutralen (z.B. Zucker) und elektrisch geladenen (Salze) niedermolekularen Substanzen. Ferner können gelöste Polymere (z.B. Proteine) und Polyelektrolyte die Flockung hervorrufen. Mit zunehmender Komplexität von Multikomponentensystemen nimmt die Wahrscheinlichkeit auftretender Inkompatibilitäten zu.

Eine besondere Herausforderung stellen in diesem Zusammenhang Getränke-Konzentrate dar, die häufig über längere Zeit (Wochen bis Monate) gelagert werden müssen, bevor daraus das gebrauchsfertige Getränk hergestellt wird. Gründe für die erhöhte Sensibilität sind die hohen Konzentrationen an Zucker und anderen Ingredientien, kombiniert mit einem niedrigen pH-Wert von 2 bis 3. Ebenfalls kritisch kann der Einsatz in sogenannten "Sportler-Getränken" sein, die in der Regel eine höhere Konzentration an destabilisierenden Salzen aufweisen.

Diese Inkompatibilitäten können zu Koaleszenz und schließlich zum Brechen von Emulsionen führen. Die Folge sind Koagulation und Ausflockung der fettlöslichen Bestandteile. So können z.B. in Getränken und Getränkekonzentraten sogenannte Ringing-Effekte, d.h. das Abscheiden von unlöslichen festen oder flüssigen Komponenten an der flüssig/Luft-Grenzfläche in Form eines Ringes sowie Flockung einschließlich Sedimentation bzw. Flotation des Koagulats auftreten.

In der Praxis können solche Probleme mühsam und nur teilweise durch technische Maßnahmen wie nachträgliche Hochdruckhomogenisierung von Getränken gelöst werden.

Aufgabe war es daher, ein Verfahren unter Verwendung möglichst breit einsetzbarer Ausgangsstoffe bereitzustellen, das Emulsionen und Suspensionen dahingehend verbessert, daß die physikalischen Instabilitäten gar nicht auftreten.

Diese Aufgabe wurde erfindungsgemäß gelöst durch ein Verfahren zur Stabilisierung von flüssigen, wäßrigen Präparaten fettlöslicher Substanzen, dadurch gekennzeichnet, daß man
a) eine schutzkolloidfreie o/w-Emulsion, enthaltend 1 bis 50 Gew.-%, bezogen auf die Gesamtmenge der o/w-Emulsion mindestens einer fettlöslichen Substanz mit mindestens einem Schutzkolloid versetzt und
b) diese Zubereitung mit einer weiteren wäßrigen Phase vermischt.

Als schutzkolloidfreie o/w-Emulsionen lassen sich in der Regel alle an sich bekannten und für den Lebensmittel- bzw. Kosmetikbereich zugelassenen, u.a. in DE-A-2 363 534, US 2,628,930, EP-A-0 361 928 und DE-A-2 411 529 beschriebenen Emulsionen verwenden

Bevorzugt werden im Rahmen des erfindungsgemäßen Verfahrens solche schutzkolloidfreien o/w-Emulsionen gemäß EP-A-0 551 638 eingesetzt, die als wesentliche Bestandteile
a₁) 1 bis 50 Gew-% mindestens einer feindispergierten physiologisch verträglichen fettlöslichen Substanz als disperse Phase,
a₂) 99 bis 50 Gew.-% Glycerin oder Glycerin im Gemisch mit Wasser als äußere Phase unter Verwendung von
a₃) 0,2 bis 5 Gew-% Estern der Ascorbinsäure mit langkettigen Fettsäuren als Emulgator und Stabilisator enthalten, wobei sich die Gew.-% Angaben jeweils auf die Gesamtmenge der o/w-Emulsion beziehen.

Als physiologisch verträgliche, fettlösliche Substanzen kommen z.B. die fettlöslichen Vitamine A, D, E oder K oder deren Derivate, beispielsweise Vitamin A- und Vitamin E-Ester wie Retinylacetat oder Tocopherylacetat, Tocotrienol, Vitamin K₁, Vitamin K₂ sowie Carotinoide, wie z.B. Canthaxanthin, Astaxanthin, Zeaxanthin, Lutein, Lycopin, Apocarotinal und insbesondere β-Carotin in Betracht. Weitere im Rahmen der Erfindung einsetzbare fettlösliche Substanzen sind polyungesättigte Fettsäuren wie z.B. Arachidonsäure, Docosahexaensäure, Eicosapentaensäure, Linolsäure, Linolensäure sowohl in freier Form als auch als Triglycerid sowie Aromaöle wie Citrusöle, beispielsweise Centaur Orange® (Fa. Givaudan Roure).

Ferner kommen Öle wie z.B. Weizenöl, Sonnenblumenöl, Naiskeimöl oder Mischungen der genannten Öle in Betracht.

Schließlich sind ganz allgemeine, beliebige fettlösliche Substanzen als Bestandteil der o/w-Emulsion geeignet, die eine physiologische Rolle im menschlichen oder tierischen Organismus spielen und aufgrund ihrer Wasserunlöslichkeit in der Regel zu Emulsionen verarbeitet werden.

Bevorzugte fettlösliche Substanzen im Rahmen der vorliegenden Erfindung sind insbesondere die o.g. fettlöslichen Vitamine, z.B. Vitamin A, D, E und K und deren Derivate, sowie die mehrfach ungesättigten Fettsäuren und β-Carotin. Besonders bevorzugt ist β-Carotin.

Die Konzentration der physiologisch verträglichen fettlöslichen Substanzen in der schutzkolloidfreien o/w-Emulsion beträgt in der Regel 1 bis 50 Gew.-%, vorzugsweise 10 bis 40 und insbesondere 20 bis 30 Gew.-%, bezogen auf die Gesamtmenge der o/w-Emulsion. Das an sich schwerlösliche β-Carotin ist jedoch in der Regel in Konzentrationen von 1 bis 20 Gew.-%, vorzugsweise 5 bis 15 Gew.-%, bezogen auf die Gesamtmenge der o/w-Emulsion enthalten.

Die Menge des Glycerins beträgt im allgemeinen 99 bis 50, vorzugsweise 80 bis 60 Gew.-%, bezogen auf die Gesamtmenge der o/w-Emulsion, wobei das Glycerin teilweise durch Wasser ersetzt sein kann im Bereich des Gewichtsverhältnisses Glycerin : Wasser von 100 : 0 bis 50 : 50.

Als Ester der Ascorbinsäure sind vor allem die Ester der Ascorbinsäure mit C₁₆- bis C₁₈-Fettsäuren zu nennen, insbesondere Ascorbylpalmitat.

Die Wirkung des Ascorbylpalmitats als Emulgator kann durch Bildung eines Salzes, in der Regel ein Alkalisalz, insbesondere Natriumsalz, noch gesteigert werden, wobei Natronlauge in der Regel in der 0,5 bis 1 molaren Menge zugesetzt wird.

Ascorbylpalmitat ist ein in der Lebens- und Futtermittelindustrie breit einsetzbarer, physiologisch unbedenklicher Emulgator, der darüber hinaus auch antioxidativ wirkt, eine Eigenschaft, die bei Carotinoiden und vielen Vitaminen die Stabilität erhöht. Der antioxidative Effekt läßt sich bekanntermaßen dadurch noch steigern, daß der fettlöslichen Phase zusätzlich Tocopherol beigemischt wird.

Die Teilchengrößen der dispersen Phase der schutzkolloidfreien o/w-Emulsion liegen unter 5µm, insbesondere unter 0,5µm.

Das erfindungsgemäße Verfahren zeichnet sich dadurch aus, daß man die eingangs beschriebene, schutzkolloidfreie o/w-Emulsion im Verfahrensschritt a) zunächst mit mindestens einem Schutzkolloid vermischt. Dabei kann das Schutzkolloid sowohl in fester Form als auch bevorzugt in Form einer wäßrigen Lösung mit der Emulsion vermischt werden. Besonders bevorzugt wird im Schritt a) die o/w-Emulsion in eine wäßrige Schutzkolloidlösung eingerührt.

Geeignete Schutzkolloide sind sowohl elektrisch geladene Polymere (Polyelektrolyte) als auch neutrale Polymere. Typische Beispiele sind u.a. Gummi Arabicum, Gelatine, Kasein, Kaseinat, Pektin, Dextrin, Johannisbrotkernmehl, Guar gum, Xanthan, oder Pflanzenproteine wie z.B. Sojaproteine, die gegebenenfalls hydrolysiert sein können und deren Mischungen. Es können aber auch Polyvinylalkohol, Polyvinylpyrrolidon, Methylcellulose, Carboxymethylcellulose, Hydroxypropylcellulose und Alginate eingesetzt werden. Bevorzugte Schutzkolloide sind Verbindungen, ausgewählt aus der Gruppe, bestehend aus Gelatine wie Rinder-, Schweine- und Fischgelatine, Pflanzenproteine, Pektin, Kasein, Kaseinat und Gummi Arabicum. Besonders bevorzugt eingesetzte Schutzkolloide sind wäßrige Lösungen von Pektin, Kasein, Kaseinat, Gummi Arabicum und/oder Fischgelatine.

Durch das Mischen der schutzkolloidfreien o/w-Emulsion mit der wäßrigen Schutzkolloidlösung erfolgt eine Verdünnung der Einulsion um den Faktor 5 bis 1000, bevorzugt 5 bis 100, besonders bevorzugt um den Faktor 10 bis 50.

Im Hinblick auf die spätere Stabilität der erfindungsgemäß hergestellten wäßrigen Präparate fettlöslicher Substanzen hat sich als vorteilhaft herausgestellt, wenn man die Verdünnung der schutzkolloidfreien o/w-Emulsion mit rein wäßrigen Lösungen der Schutzkolloide ohne weitere Zusätze durchführt.

Die Konzentration der Schutzkolloide in der wäßrigen Lösung liegt im Bereich von 0,1 bis 10 Gew.-%, bevorzugt 0,2 bis 5 Gew.-%, besonders bevorzugt im Bereich von 0.5 bis 2 Gew.-%.

Die so erhaltene verdünnte, schutzkolloidhaltige o/w-Emulsion kann anschließend im Verfahrensschritt b) mit einer weiteren wäßrigen Phase vermischt werden.

Hierbei kann es sich um ein wäßriges Multikomponentensystem, beispielsweise um eine wäßrige Lebensmittelzubereitung handeln. Insbesondere sind in diesem Zusammenhang Getränkegrundstoffe, beispielsweise wäßrige Mineralsalzmischungen oder wäßrige Vitaminmischungen zu nennen.

Durch das erfindungsgemäße Verfahren lassen sich in aller Regel auch kritische Systeme wie Konzentrate oder isotonische Getränke, die einen nicht unerheblichen Anteil an verschiedenen Salzen aufweisen, herstellen, ohne daß es zu unerwünschten Effekten wie Ringbildung, Flockung oder Farbverlust infolge Koagulation der dispersen Phase kommt.

Je nach Anwendungsgebiet können den erfindungsgemäß hergestellten wäßrigen Präparaten noch weitere Komponenten/Zusatzstoffe zugegeben werden und falls erforderlich technische Schritte wie Pasteurisation und Homogenisation mit den Präparaten durchgeführt werden.

Das Vermischen der schutzkolloidfreien o/w-Emulsion mit den Schutzkolloiden kann unmittelbar vor der Zugabe der Emulsion zur wäßrigen Lebensmittelzubereitung erfolgen. Es ist aber auch möglich, die im Verfahrensschritt a) gebildete, schutzkolloidhaltige Emulsion nach ihrer Herstellung zunächst abzufüllen und zu lagern um sie erst bei Bedarf zur wäßrigen Lebensmittelzubereitung zu geben.

Im Vergleich zum erfindungsgemäßen Verfahren hat sich gezeigt, daß durch eine separate nachträgliche Zugabe der Schutzkolloide zu einer bereits mit schutzkolloidfreien o/w-Emulsionen versetzten Lebensmittelzubereitung, insbesondere Getränkezubereitungen keine stabilisierende Wirkung hinsichtlich Ringbildung, Flockung oder Koagulation erzielt wird.

Gegenstand der Erfindung sind auch stabile, flüssige Präparate fettlöslicher Substanzen die nach dem oben beschriebenen Verfahren erhältlich sind.

Bevorzugt handelt es sich bei diese Präparaten um flüssige Lebensmittelzubereitungen, insbesondere um Getränke, die als fettlösliche Substanzen die bereits oben genannten Vitamine, Carotinoide, Fettsäuren, Aromastoffe und Gemische davon enthalten.

Besonders bevorzugt sind solche der o.g. Präparate, enthaltend β-Carotin in einer Konzentration im Bereich von 1 bis 100 ppm, bevorzugt 2 bis 50 ppm, besonders bevorzugt 5 bis 20 ppm.

Anhand der folgenden Beispiele soll das erfindungsgemäße Verfahren näher erläutert werden.

### Beispiel 1:

Herstellung eines β-Carotin-haltigen Getränkekonzentrates für Limonade

Von einer gemäß EP-A-0 557 638 hergestellten 10 Gew.-%igen β-Carotin-Emulsion wurden 4,2 g in 56 g folgender Medien eingerührt:
a) in voll-entsalztem (VE) Wasser,
b) in Trinkwasser,
c) in VE-Wasser, in dem 1 Gew.-% an Gummi Arabicum gelöst war
und dann einem Getränkegrundstoff folgender Zusammensetzung beigemischt:
- 5,3 g: Johannisbrotkernmehl (vorgequollen in 70 g Trinkwasser, 30 Min. bei 95°C),
- 211,5 g: Citronensäure,
- 830 g: Orangenkonzentrat (1 : 5),
- 4 g: Orangenaroma (Centaur Orange, Fa. Givaudan)

Der Getränkegrundstoff wurde mittels Hochdruckhomogenisator 2 mal bei 180 bar homogenisiert.

Zur Herstellung des Getränke-Konzentrates wurde folgendermaßen verfahren:

4,2 kg Zucker und 12 g Ascorbinsäure wurden in 7 kg Trinkwasser gelöst und mit 680 g des β-Carotin-haltigen Getränke-Grundstoffes vermischt. Das Getränke-Konzentrat wurde anschließend in einer Kurzzeiterhitzungsanlage bei 92°C 60 Sekunden pasteurisiert.

Im Falle von 1a) und 1b) kam es im Verlauf einer einwöchigen Lagerung des Konzentrates zu Ringbildung der färbenden Komponente. Bei nachträglicher Zugabe einer entsprechenden Menge an Gummi Arabicum verschwand die Flockung nicht.

Im Falle von 1c) trat auch nach mehrwöchiger Lagerung keine physikalische Instabilität auf.

### Beispiel 2:

Herstellung eines Getränke-Konzentrates mit Vitamin A

Es wurde eine 1 : 100 Verdünnung von einer gemäß EP-A-0 557 638 hergestellten 10 %igen Vitamin A-Palmitat-Emulsion hergestellt und analog zu Beispiel 1 weiterverarbeitet.

Die kolloidale Stabilität war identisch mit den Ergebnissen aus Beispiel 1.

### Beispiel 3:

Herstellung eines β-Carotin-haltigen Sportler-Getränkes

Von einer gemäß EP-A-0 557 638 hergestellten 10 %igen β-Carotin-Emulsion wurden 0,04 g in 50 g folgender Medien eingerührt:
3a) in voll-entsalztem (VE) Wasser
3b) in Trinkwasser
3c) in VE-Wasser, in dem 1 Gew.-% an Pektin gelöst war.

Die Lösungen wurden jeweils zu folgender Mischung gegeben:
- 105 g: Zucker
- 7,5 g: Citronensäure
- 0,2 g: Vitamin C
- 5,2 g: Mineralsalzmischung (aus 9,5 Teile KCl; 21,3 Teile Trinatrium-citrat; 8,6 Teile Calciumihydrogen-phosphat; 12,3 Teile Trimagnesium-dicitrat x 9 H₂0)
- 1,5 g: wasserlösliches Orangenaroma ("Orange 503985 T", Fa. Firmenich, Kerpen)
- ad 1000 g: Trinkwasser.

Nach 4-wöchiger Lagerung der in 200 ml Glasflaschen abgefüllten Getränke bei Raumtemperatur zeigten die Proben 3a) und 3b) nur noch geringe Farbstärke infolge Koagulation der β-Carotin-haltigen Tröpfchen. Probe 3c) hingegen hatte sich farblich nicht verändert und wies keinerlei Ringbildung oder Koagulation auf.

## Patentansprüche

1. Verfahren zur Stabilisierung von flüssigen, wäßrigen Präparaten fettlöslicher Substanzen, dadurch gekennzeichnet, daß man
a) eine schutzkolloidfreie o/w-Emulsion, enthaltend 1 bis 50 Gew.-%, bezogen auf die Gesamtmenge der o/w-Emulsion mindestens einer fettlöslichen Substanz mit mindestens einem Schutzkolloid versetzt und
b) diese Zubereitung mit einer weiteren wäßrigen Phase vermischt.

2. Verfahren nach Anspruch 1, dadurch gekennzeichnet, daß die in der schutzkolloidfreien o/w-Emulsion enthaltenen fettlöslichen Substanzen in Teilchengrößen kleiner 5 µm vorliegen.

3. Verfahren nach einem der Ansprüche 1 oder 2, dadurch gekennzeichnet, daß die unter a) genannte schutzkolloidfreie o/w-Emulsion als wesentliche Bestandteile
a₁) 1 bis 50 Gew-% mindestens einer feindispergierten physiologisch verträglichen fettlöslichen Substanz als disperse Phase,
a₂) 99 bis 50 Gew.-% Glycerin oder Glycerin im Gemisch mit Wasser als äußere Phase unter Verwendung von
a₃) 0,2 bis 5 Gew-% Estern der Ascorbinsäure mit langkettigen Fettsäuren als Emulgator und Stabilisator enthält, wobei sich die Gew.-% Angaben jeweils auf die Gesamtmenge der o/w-Emulsion beziehen.

4. Verfahren nach einem der Ansprüche 1 bis 3, dadurch gekennzeichnet, daß man die unter a) genannte o/w-Emulsion zunächst mit einer wäßrigen Lösung mindestens eines Schutzkolloids verdünnt und die so erhaltene verdünnte schutzkolloidhaltige o/w-Emulsion im Verfahrensschritt b) mit einer weiteren wäßrigen Phase vermischt.

5. Verfahren nach Anspruch 4, dadurch gekennzeichnet, daß im Verfahrensschritt a) eine Verdünnung der o/w-Emulsion um den Faktor 5 bis 1000 erfolgt.

6. Verfahren nach einem der Ansprüche 1 bis 5, dadurch gekennzeichnet, daß es sich bei den flüssigen, wäßrigen Präparaten um fettlösliche Substanzen enthaltende Getränke handelt.

7. Verfahren nach einem der Ansprüche 1 bis 6, dadurch gekennzeichnet, daß es sich bei den im Verfahrensschritt a) eingesetzten Schutzkolloiden um Verbindungen, ausgewählt aus der Gruppe, bestehend aus Gelatine, Pflanzenproteine, Pektin, Kasein, Kaseinat und Gummi Arabicum handelt.

8. Verfahren nach einem der Ansprüche 1 bis 7, dadurch gekennzeichnet, daß es sich bei den in der o/w-Emulsion enthaltenen fettlöslichen Substanzen um Verbindungen, ausgewählt aus der Gruppe, bestehend aus Vitamin A, D, E oder K oder deren Derivaten, Carotinoiden, polyungesättigten Fettsäuren und Aromaöle handelt.

9. Stabile, flüssige Präparate fettläslicher Substanzen erhältlich nach einem Verfahren gemäß einem der Ansprüche 1 bis 8.

10. Stabile, flüssige Präparate nach Anspruch 9, enthaltend β-Carotin in einer Konzentration im Bereich von 1 bis 100 ppm.

11. Stabile, flüssige Präparate nach einem der Ansprüche 9 oder 10, dadurch gekennzeichnet, daß es sich um ein Getränk handelt.
